# EUROPEAN PATENT SPECIFICATION

(11) **EP 0 920 873 B1**
(45) Date of publication and mention of the grant of the patent: **25.02.2004**
(21) Application number: 98123225.9
(22) Date of filing: 07.12.1998
(51) Int. Cl.: A61K 38/29, A61K 9/08, A61K 47/10, A61K 47/26, A61P 19/10

(54) **Stabilized teriparatide solutions**
Teriparatidhaltige stabilisierte Lösungen
Solutions stabilisées de Tériparatide

(30) Priority: 09.12.1997 US 69075 P
(43) Date of publication of application: 09.06.1999
(60) Divisional application: 03104219.5
(73) Proprietor: ELI LILLY AND COMPANY, Indianapolis, Indiana 46285 (US)
(72) Inventor: Chang, Chin-Ming, Fishers, Indiana 46038 (US); Havel, Henry A., Indianapolis, indiana 46220 (US)
(74) Representative: Burnside, Ivan John

(56) References cited:
- EP-A- 0 302 772
- EP-A- 0 619 119
- WO-A-91/06564
- WO-A-95/17207
- DATABASE WPI Week 8817 Derwent Publications Ltd., London, GB; AN 88-115396 [17] XP002102409 & JP 63 060940 A (TOYO JOZO)
- CHEMICAL ABSTRACTS, vol. 111, no. 26, 25 December 1989 Columbus, Ohio, US; abstract no. 239514, XP002105393 & JP 01 016799 A (TOYO JOZO)

### SEQUENCE LISTING

<110> Eli Lilly and Company
<120> STABILIZED TERIPARATIDE SOLUTIONS
<130> 3797.16WO01
<140> NEW FILING
   <141> 1998-12-08
<150> 60/069,075
   <151> 1997-12-09
<160> 2
<170> PatentIn Ver. 2.0
<210> 1
   <211> 84
   <212> PRT
   <213> Homo sapiens
<400> 1
   Ser Val Ser Glu Ile Gln Leu Met His Asn Leu Gly Lys His Leu Asn
     1               5                  10                  15
   Ser Met Glu Arg Val Glu Trp Leu Arg Lys Lys Leu Gln Asp Val His
                20                  25                  30
   Asn Phe Val Ala Leu Gly Ala Pro Leu Ala Pro Arg Asp Ala Gly Ser
            35                  40                  45
   Gln Arg Pro Arg Lys Lys Glu Asp Asn Val Leu Val Glu Ser His Glu
        50                  55                  60
   Lys Ser Leu Gly Glu Ala Asp Lys Ala Asn Val Asp Val Leu Thr Lys
    65                  70                  75                  80
   Ala Lys Ser Gln
<210> 2
   <211> 34
   <212> PRT
   <213> Homo sapiens
<400> 2
   Ser Val Ser Glu Ile Gln Leu Met His Asn Leu Gly Lys His Leu Asn
     1               5                  10                  15
   Ser Met Glu Arg Val Glu Trp Leu Arg Lys Lys Leu Gln Asp Val His
                20                  25                  30
   Asn Phe

## Description

### TECHNICAL FIELD

This invention relates to pharmaceutical compositions containing a parathyroid hormone. More particularly, the invention relates to teriparatide, PTH(1-34), stabilized solution formulations

### BACKGROUND OF THE INVENTION

Parathyroid hormone (PTH) is a secreted, 84 amino acid product of the mammalian parathyroid gland that controls serum calcium levels through its action on various tissues, including bone. Studies in humans with certain forms of PTH have demonstrated an anabolic effect on bone, and have prompted significant interest in its use for the treatment of osteoporosis and related bone disorders.

Using the N-terminal 34 amino acids of the bovine and human hormone for example, which by all published accounts are deemed biologically equivalent to the full length hormone, it has been demonstrated in humans that parathyroid hormone enhances bone growth particularly when administered in pulsatile fashion by the subcutaneous route. A slightly different form of PTH, human PTH(1-38) has shown similar results.

PTH preparations have been reconstituted from fresh or lyophilized hormone, and incorporate various forms of carrier, excipient and vehicle. Most are prepared in water-based vehicles such as saline, or water acidified typically with acetic acid to solubilize the hormone. The majority of reported formulations also incorporate albumin as a stabilizer (see for example Reeve at al., Br. Med. J., 1980, 280:6228; Reeve at al., Lancet, 1976, 1:1035; Reeve at al., Calcif. Tissue Res., 1976, 21:469; Hodsman et al., Bone Miner; 1990, 9(2):137; Tsai et al., J. Clin. Endocrinol Metab., 1989, 69(5):1024; Isaac et al., Horm. Metab. Res., 1980, 12(9):487; Law et al., J. Clin Invest. 1983, 72(3):1106; and Hulter, J. Clin Hypertens, 1986, 2(4):360). Other reported formulations have incorporated an excipient such as mannitol, which is present either with the lyophilized hormone or in the reconstitution vehicle. Formulations representative of those employed for human studies include a human PTH(1-34) (SEQ ID NO: 2) preparation consisting, upon reconstitution, of mannitol, heat inactivated human serum albumin, and caproic acid (a protease inhibitor) as absorption enhancer (see Reeve at al., 1976, Calcif. Tissue Res., 21, Suppl., 469-477); a human PTH(1-38) preparation reconstituted into a saline vehicle (see Hodsman et al., 1991, 14(1), 67-83); and a bovine PTH(1-34) preparation in aqueous vehicle pH adjusted with acetic acid and containing albumin. There is also an International Reference preparation which for human PTH (1-84) (SEQ ID NO: 1) consists of 100 ng of hormone ampouled with 250 µg human serum albumin and 1.25 mg lactose (1981), and for bovine PTH (1-84) consists of 10 µg lyophilized hormone in 0.01M acetic acid and 0.1% w/v mannitol (see Martindale, The Extra Pharmacoepia, The Pharmaceutical Press, London, 29th Edition, 1989 at p. 1338).

A recent attempt at improving the stability for the lyophilized preparation of h-PTH(1-34) (SEQ ID NO: 2) is reported in EP 619 119 with a combination of sugar and sodium chloride. Also U.S. Pat. No. 5,496,801 describes a freeze-dried composition for the natural hormone, PTH(1-84), containing mannitol as an excipient and a citrate source as a non-volatile buffering agent.

Commercial exploitation of parathyroid hormone requires the development of a formulation that is acceptable in terms of storage stability and ease of preparation. Because it is a protein and thus far more labile than the traditionally small molecular weight drugs, however, the formulation of parathyroid hormone presents challenges not commonly encountered by the pharmaceutical industry. Furthermore, like other proteins that have been formulated successfully, PTH is particularly sensitive to oxidation, deamidation and hydrolysis, and requires that its N-terminal and C-terminal sequences remain intact in order to preserve bioactivity.

It is an object of the present invention to provide a pharmaceutically useful PTH preparation, particularly one comprising, as active ingredient, teriparatide, PTH(1-34) (SEQ ID NO: 2).

### SUMMARY OF THE INVENTION

The present invention provides a pharmaceutical composition in the form of a stabilized solution containing a parathyroid hormone (PTH) in a therapeutically effective amount The solution is storage stable and, in sterile form, may be stored in vials or cartridges ready for parenteral administration in human patients. The advantages of the present solution is the elimination of the need for lyophilization.

Accordingly, the present invention is a parathyroid hormone solution including:
(a) a therapeutically effective amount of a parathyroid hormone;
(b) an effective amount of a stabilizing agent;
(c) a buffering agent in an amount sufficient to maintain the pH of the composition within a range of about 3-7; and
(d) the balance being water.

This solution may, if desired, undergo lyophilization to form a freeze-dried powder containing not more than 2% water by weight.

Another aspect of the present invention is a parathyroid hormone solution including:
(a) a therapeutically effective amount of a parathyroid hormone;
(b) from about 1 to 20 wt-% of a stabilizing agent;
(c) a buffering agent in an amount sufficient to maintain the pH of the composition within a range of about 3-7 and selected from an acetate or tartrate source;
(d) from about 0.1 to 2 wt-% of a parenterally acceptable preservative; and
(e) the balance being water.

### DETAILED DESCRIPTION

The invention relates to parathyroid hormone solutions that exhibit storage stability in terms of hormone composition and activity.

As active ingredient, the composition or solution may incorporate the full length, 84 amino acid form of parathyroid hormone, particularly the human form, hPTH (1 -84) (SEQ ID NO: 1), obtained either recombinantly, by peptide synthesis or by extraction from human fluid. See, for example, U.S. Pat. No. 5,208,041. The amino acid sequence for hPTH (1-84) is reported by Kimura et al. in Biochem. Biophys. Res. Comm., 114(2):493 (SEQ ID NO: 1).

The composition or solution may also incorporate as active ingredient fragments or variants of fragments of human PTH or of rat, porcine or bovine PTH that have human PTH activity as determined in the ovarectomized rat model of osteoporosis reported by Kimmel et al., Endocrinology, 1993, 32(4):1577.

The parathyroid hormone fragments desirably incorporate at least the first 34 N-terminal residues, such as PTH(1-34) (SEQ ID NO: 2), PTH(1-37), PTH(1-38) and PTH(1-41). Alternatives in the form of PTH variants incorporate from 1 to 5 amino acid substitutions that improve PTH stability and half-life, such as the replacement of methionine residues at positions 8 and/or 18 with leucine or other hydrophobic amino acid that improves PTH stability against oxidation and the replacement of amino acids in the 25-27 region with trypsin-insensitive amino acids such as histidine or other amino acid that improves PTH stability against protease. These forms of PTH are embraced by the term "parathyroid hormone" as used generically herein. The preferred hormone is human PTH(1-34) (SEQ ID NO: 2) also known as teriparatide. The hormones may be obtained by known recombinant or synthetic methods, such as described in U.S. Pat. No. 4,086,196.

The stabilizing agent incorporated into the solution or composition includes a polyol which includes a saccharide, preferably a monosaccharide or disaccharide, e.g., glucose, trehalose, raffinose, or sucrose; a sugar alcohol such as, for example, mannitol, sorbitol or inositol, and a polyhydric alcohol such as glycerine or propylene glycol or mixtures thereof. A preferred polyol is mannitol or propylene glycol. The concentration of polyol may range from about 1 to about 20 wt-%, preferably about 3 to 10 wt-% of the total solution.

The buffering agent employed in the solution or composition of the present invention may be any acid or salt combination which is pharmaceutically acceptable and capable of maintaining the aqueous solution at a pH range of 3 to 7, preferably 3-6. Useful buffering systems are, for example, acetate, tartrate or citrate sources. Preferred buffer systems are acetate or tartrate sources, most preferred is an acetate source. The concentration of buffer may be in the range of about 2 mM to about 500 mM, preferably about 2 mM to 100 mM.

The stabilized solution or composition of the present invention may also include a parenterally acceptable preservative. Such preservatives include, for example, cresols, benzyl alcohol, phenol, benzalkonium chloride, benzethonium chloride, chlorobutanol, phenylethyl alcohol, methyl paraben, propyl paraben, thimerosal and phenylmercuric nitrate and acetate. A preferred preservative is m-cresol or benzyl alcohol; most preferred is m-cresol. The amount of preservative employed may range from about 0.1 to about 2 wt-%, preferably about 0.3 to about 1.0 wt-% of the total solution.

Thus, the present invention has provided, for example, a stabilized teriparatide solution containing mannitol, acetate and m-cresol with a predicted shelf-life of over 15 months at 5°C.

The PTH solution and composition of the present invention incorporate PTH in a medically effective amount, a term used with reference to amounts useful either therapeutically or in medical diagnosis. The particular amount of parathyroid hormone incorporated in the preparation can be pre-determined based on the type of PTH selected and on the intended end-use of the preparation. In one application, the preparations are exploited for therapeutic purposes, and particularly for the treatment of osteoporosis. Osteoporosis therapy entails administration of the reconstituted preparation by injection, desirably subcutaneous injection, in unit doses that reflect the prescribed treatment regimen but are, by way of example, for human PTH(1-34) (SEQ ID NO: 2), within the range from 25 µg PTH/mL of injected solution to 1000 µg/mL of injected solution per patient, with injection volumes being desirably from 0.02 to 1.3 mL. Accordingly, the purified PTH is desirably incorporated with the buffering agent and excipient to form an aqueous solution containing PTH in a concentration range from 25 µg/mL to 1000 µg/mL, preferably 100 µg/mL to 500 µg/mL, which is then sterile-filtered and filled into a vial or cartridge for use.

Once the preparation is obtained as an aqueous solution containing desired amounts and concentrations of the buffering agent, excipient and PTH, individual vials are filled with the solution to the desired volume. The advantage of the present invention is that the above solution may be prepared with sterile water without the need to undergo a freeze-drying process.

In addition to their therapeutic use, the present PTH composition can be formulated and administered to aid in medical diagnosis, and particularly to assist in establishing the diagnosis of hypoparathyroidism and pseudohypoparathyroidism in hypocalcemic patients. Except for the dose of PTH, the composition of the PTH preparation will remain as described herein for therapeutic use. An intravenously infused, single dose of human PTH(1-34) (SEQ ID NO: 2) that is equal to 200 International Units of PTH activity is appropriate for this diagnostic purpose. Diagnosis is then made by determining the effect of administered PTH or urinary cAMP levels, with cAMP elevation being indicative of the hypoparathyroidism condition, rather than its pseudoform.

The examples which follow are illustrative of the invention and are not intended to be limiting.

### EXAMPLES

### Example 1

0.1 mg rhPTH (1-34) (SEQ ID NO: 2), 50 mg mannitol, 2.5 mg m-cresol, 0.52 mg acetic acid and 0.12 mg sodium acetate were mixed into a solution with 1 ml of distilled water.

### Example 2

0.25 mg rhPTH (1-34) (SEQ ID NO: 2), 45.4 mg mannitol , 3 mg m-cresol, 0.41 mg acetic acid and 0.1 mg sodium acetate were mixed into a solution with I ml of distilled water.

The formulations of the present invention, Examples 1 and 2 were compared to solutions containing no stabilizer, 0.9% NaCl, 20 mM acetate and 10 mM acetate as primary stabilizer. The stability was measured by determining the amount in % of rhPTH (1-34) (SEQ ID NO: 2) remaining after a certain time. The measurement was made by HPLC. The results are shown in Tables 1 and 2.

**Table 1**

| Effect of Primary Stabilizer on Chemical Stability of rhPTH (1-34) at 50°C | | | | |
|---|---|---|---|---|
| | Water | 0.9% NaCl | 20 mM acetate | 10 mM acetate |
| Time, days | % Remaining | | | |
| Initial | 100 | 100 | 100 | 100 |
| 7 | 74 | 81 | 84 | 80 |
| 14 | 55 | 58 | 67 | 71 |

**Table 2**

| Comparison of Stability of rhPTH (1-34) at 30°C | | | | |
|---|---|---|---|---|
| | 20 mM acetate | 10 mM acetate | Example 1 | Example 2 |
| Time, days | % Remaining | | | |
| Initial | 100 | 100 | 100 | 100 |
| 7 | 96 | 94 | 100 | -- |
| 14 | 94 | 92 | 96 | 100 |
| 21 | 90 | 93 | 97 | -- |
| 30 | -- | 81 | 96 | 96 |

### Example 3

The following experiment was carried out to show that lyophilized powder formulations prepared from stabilized solutions of the present invention are more stable than a control which was prepared from PTH(1-34) and mannitol alone.

A control solution and solutions for samples A through O were prepared as previously described with the ingredients and concentrations shown in Table 3. The solutions were then freeze-dried and the resulting lyophilized powder formulations were stored at 40°C for a one month period. The amount of PTH(1-34) remaining in each sample was then measured by HPLC. The results are shown in Table 3.

**Table 3**

| Stability of PTH(1-34) Lyophilized Formulations at 40°C for One Month | | | | | | |
|---|---|---|---|---|---|---|
| Sample | PTH(1-34) mg/mL | Bulking Agent | Bulking Agent Conc. mg/mL | Buffer | Buffer Conc.mM | % PTH Remaining |
| Control | 0.2 | mannitol | 40 | -- | -- | 78 |
| A | 0.5 | mannitol | 30 | acetate | 5 | 90 |
| B | 0.5 | glycine | 30 | acetate | 5 | 98 |
| C | 0.5 | sucrose | 30 | acetate | 5 | 98 |
| D | 0.5 | trehalose | 30 | acetate | 5 | 97 |
| E | 0.5 | raffinose | 30 | acetate | 5 | 99 |
| F | 0.75 | mannitol | 30 | tartrate | 15 | 95 |
| G | 1.5 | sucrose & mannitol | 5/25 | tartrate | 5 | 99 |
| H | 0.75 | sucrose & mannitol | 5/25 | tartrate | 15 | 99 |
| I | 1.5 | mannitol | 30 | tartrate | 5 | 96 |
| J | 1.5 | sucrose | 30 | tartrate | 15 | 100 |
| K | 1.5 | mannitol | 30 | tartrate | 15 | 99 |
| L | 0.75 | sucrose | 30 | tartrate | 15 | 100 |
| M | 0.75 | sucrose | 30 | tartrate | 5 | 100 |
| N | 1.5 | sucrose & mannitol | 5/25 | tartrate | 15 | 99 |
| O | 1.5 | sucrose & mannitol | 5/25 | acetate | 5 | 91 * |

| | | | | | | |
|---|---|---|---|---|---|---|
| * the stability at 2 months was 96% | | | | | | |

## Claims

1. A non freeze-dried pharmaceutical composition in the form of a solution, which comprises human parathyroid hormone, a buffer to maintain a pH from greater than 3 to 7 and a stabilising agent, said solution being ready for parenteral administration in a human patient.

2. A pharmaceutical composition according to claim 1 wherein said buffer is selected from citrate, tartrate or acetate.

3. A pharmaceutical composition according to claim I or claim 2 wherein said stabilising agent is mannitol.

4. A pharmaceutical composition according to any one of claims 1 to 3, said solution further comprising water.

5. A pharmaceutical composition according to any one of the preceding claims wherein said human parathyroid hormone is selected from PTH(1-34), PTH(1-37), PTH(1-38), PTH(1-41) and PTH(1-84).

6. A pharmaceutical composition according to claim 5, wherein said human parathyroid hormone is PTH(1-34).

7. A process for preparing a vial or cartridge containing a non freeze-dried liquid pharmaceutical composition ready for parenteral administration according to any one of claims 1 to 6 comprising:
(a) combining human parathyroid hormone, a buffer to maintain a pH from greater than 3 to 7 and a stabilising agent, thereby forming a solution, and
(b) filling the vial or cartridge with said solution, from which a therapeutically effective dose of parathyroid hormone can be withdrawn for use by a patient.

8. A process for preparing a non freeze-dried pharmaceutical composition in the form of a solution comprising admixing human parathyroid hormone, a buffer to maintain a pH from 3 to 7 and a stabilising agent, said solution being ready for parenteral administration in a human patient.

9. A non freeze-dried pharmaceutical composition prepared by the process of claim 8.

10. A pharmaceutical composition according to claim 9 wherein said buffer is selected from citrate, tartrate or acetate.

11. A pharmaceutical composition according to claim 9 or claim 10 wherein said stabilising agent is mannitol.

12. A pharmaceutical composition according to any one of claims 9 to I 1 wherein said solution further comprises water.

13. A pharmaceutical composition according to any one of claims 9 to 12 wherein said human parathyroid hormone is selected from PTH(1-34), PTH(1-37), PTH(1-38), PTH(1-41) and PTH(1-84).

14. A pharmaceutical composition according to claim 13 wherein said human parathyroid hormone is PTH(1-34).

15. A vial or cartridge containing a non freeze-dried pharmaceutical composition in the form of a solution ready for parenteral administration in a human patient, said composition comprising:
(a) human parathyroid hormone;
(b) a buffer to maintain a pH from greater than 3 to 7; and
(c) a stabilising agent.

16. A vial or cartridge of claim 15 wherein said buffer is selected from citrate, tartrate or acetate.

17. A vial or cartridge according to claim 15 or claim 16 wherein said stabilising agent is mannitol.

18. A vial or cartridge according to any one of claims 15 to 17 wherein said solution further comprises water.

19. A vial or cartridge according to any one of claims 15 to 18, wherein said human parathyroid hormone is selected from the group consisting of PTH(1-34), PTH(1-37), PTH(1-38), PTH(1-41) and PTH(1-84).

20. A vial or cartridge according to claim 19 wherein said human parathyroid hormone is PTH(1-34).

21. A vial or cartridge according to any one of claims 15 to 20 wherein said parathyroid hormone is at a concentration of 25 µg/ml to 1000 µg/ml.

22. A non freeze-dried pharmaceutical composition according to any one of claims 1 to 6 or any one of claims 9 to 14 for use as a medicament.

23. A non freeze-dried pharmaceutical composition according to claim 22 for use in the treatment of osteoporosis.

24. Use of a non freeze-dried pharmaceutical composition according to any one of claims 1 to 6 or any one of claims 9 to 14 in the manufacture of a medicament.

25. Use according to claim 24 in the manufacture of a medicament to treat osteoporosis.

## Patentansprüche

1. Nicht gefriergetrocknete pharmazeutische Zusammensetzung in Form einer Lösung, umfassend ein Humanparathormon, einen Puffer zur Aufrechterhaltung eines pH-Wertes von größer als 3 bis 7 und einen Stabilisator, wobei diese Lösung fertig ist für eine parenterale Verabreichung an einen Menschen als Patienten.

2. Pharmazeutische Zusammensetzung nach Anspruch 1, worin der Puffer ausgewählt ist aus einem Citrat, Tartrat oder Acetat.

3. Pharmazeutische Zusammensetzung nach Anspruch 1 oder 2, worin der Stabilisator Mannit ist.

4. Pharmazeutische Zusammensetzung nach einem der Ansprüche 1 bis 3, worin die Lösung ferner auch Wasser enthält.

5. Pharmazeutische Zusammensetzung nach einem der vorhergehenden Ansprüche, worin das Humanparathormon ausgewählt ist aus PTH(1-34), PTH(1-37), PTH(1-38), PTH(1-41) und PTH(1-84).

6. Pharmazeutische Zusammensetzung nach Anspruch 5, worin das Humanparathormon PTH(1-34) ist.

7. Verfahren zur Herstellung eines Fläschchens oder einer Patrone, die eine nicht gefriergetrocknete flüssige pharmazeutische Zusammensetzung enthält, welche fertig ist für eine parenterale Verabreichung, gemäß einem der Ansprüche 1 bis 6, durch
(a) Kombination eines Humanparathormons, eines Puffers zur Aufrechterhaltung eines pH-Wertes von größer als 3 bis 7 und eines Stabilisators unter Bildung einer Lösung und
(b) Füllung des Fläschchens oder der Patrone mit dieser Lösung, woraus eine therapeutisch wirksame Dosis an Parathormon zur Anwendung durch einen Patienten entnommen werden kann.

8. Verfahren zur Herstellung einer nicht gefriergetrockneten pharmazeutischen Zusammensetzung in Form einer Lösung durch Vermischung eines Humanparathormons, eines Puffers zur Aufrechterhaltung eines pH-Wertes von 3 bis 7 und eines Stabilisators, wobei diese Lösung fertig ist für eine parenterale Verabreichung an einen Menschen als Patienten.

9. Nicht gefriergetrocknete pharmazeutische Zusammensetzung, hergestellt nach dem Verfahren von Anspruch 8.

10. Pharmazeutische Zusammensetzung nach Anspruch 9, worin der Puffer ausgewählt ist aus einem Citrat, Tartrat oder Acetat.

11. Pharmazeutische Zusammensetzung nach Anspruch 9 oder 10, worin der Stabilisator Mannit ist.

12. Pharmazeutische Zusammensetzung nach einem der Ansprüche 9 bis 11, worin die Lösung ferner auch Wasser enthält.

13. Pharmazeutische Zusammensetzung nach einem der Ansprüche 9 bis 12, worin das Humanparathormon ausgewählt ist aus PTH(1-34), PTH(1-37), PTH(1-38), PTH(1-41) und PTH(1-84).

14. Pharmazeutische Zusammensetzung nach Anspruch 13, worin das Humanparathormon PTH(1-34) ist.

15. Fläschchen oder Patrone, die eine nicht gefriergetrocknete pharmazeutische Zusammensetzung in Form einer Lösung enthält, welche fertig ist für eine parenterale Verabreichung an einen Menschen als Patienten, wobei diese Zusammensetzung umfasst
(a) ein Humanparathormon,
(b) einen Puffer zur Aufrechterhaltung eines pH-Wertes von größer als 3 bis 7 und
(c) einen Stabilisator.

16. Fläschchen oder Patrone nach Anspruch 15, worin der Puffer ausgewählt ist aus einem Citrat, Tartrat oder Acetat.

17. Fläschchen oder Patrone nach Anspruch 15 oder 16, worin der Stabilisator Mannit ist.

18. Fläschchen oder Patrone nach einem der Ansprüche 15 bis 17, worin die Lösung ferner auch Wasser enthält.

19. Fläschchen oder Patrone nach einem der Ansprüche 15 bis 18, worin das Humanparathormon ausgewählt ist aus der Gruppe bestehend aus PTH(1-34), PTH(1-37), PTH(1-38), PTH(1-41) und PTH(1-84).

20. Fläschchen oder Patrone nach Anspruch 19, worin das Humanparathormon PTH(1-34) ist.

21. Fläschchen oder Patrone nach einem der Ansprüche 15 bis 20, worin das Parathormon in einer Konzentration von 25 µg/ml bis 1.000 mg/ml vorhanden ist.

22. Nicht gefriergetrocknete pharmazeutische Zusammensetzung nach einem der Ansprüche 1 bis 6 oder einem der Ansprüche 9 bis 14 zur Verwendung als Arzneimittel.

23. Nicht gefriergetrocknete Zusammensetzung nach Anspruch 22 zur Verwendung bei der Behandlung von Osteoporose.

24. Verwendung einer nicht gefriergetrockneten pharmazeutischen Zusammensetzung nach einem der Ansprüche 1 bis 6 oder einem der Ansprüche 9 bis 14 zur Herstellung eines Arzneimittels.

25. Verwendung nach Anspruch 24 zur Herstellung eines Arzneimittels zur Behandlung von Osteoporose.

## Revendications

1. Composition pharmaceutique non lyophilisée sous forme d'une solution, laquelle comprend de l'hormone parathyroïdienne humaine, un tampon destiné à maintenir un pH compris entre 3 et 7 et un agent stabilisateur, ladite solution étant prête à être administrée par voie parentérale à un patient humain.

2. Composition pharmaceutique selon la revendication 1, dans laquelle ledit tampon est choisi parmi un citrate, un tartrate ou un acétate.

3. Composition pharmaceutique selon la revendication 1 ou la revendication 2, dans laquelle ledit agent stabilisateur est du mannitol.

4. Composition pharmaceutique selon l'une quelconque des revendications 1 à 3, ladite solution comprenant en outre de l'eau.

5. Composition pharmaceutique selon l'une quelconque des revendications précédentes, dans laquelle ladite hormone parathyroïdienne humaine est choisie parmi PTH(I-34), PTH(I-37), PTH(I-38), PTH(I-41) et PTH(I-84).

6. Composition pharmaceutique selon la revendication 5, dans laquelle ladite hormone parathyroïdienne humaine est PTH(I-34).

7. Procédé pour la préparation d'un flacon ou d'une ampoule contenant une composition pharmaceutique liquide non lyophilisée prête à être administrée par voie parentérale, selon l'une quelconque des revendications 1 à 6 comprenant
(a) la combinaison d'hormone parathyroïdienne humaine, d'un tampon destiné à maintenir un pH compris entre 3 et 7 et d'un agent stabilisateur pour former une solution, et
(b) le remplissage du flacon ou de l'ampoule avec ladite solution, duquel une dose d'hormone parathyroïdienne thérapeutiquement efficace peut être extraite pour administration à un patient.

8. Procédé pour la préparation d'une composition pharmaceutique non lyophilisée sous forme d'une solution comprenant le mélange d'hormone parathyroïdienne humaine, d'un tampon destiné à maintenir un pH compris entre 3 à 7 et d'agent stabilisateur, ladite solution étant prête à être administrée par voie parentérale à un patient humain.

9. Composition pharmaceutique non lyophilisée préparée selon le procédé de la revendication 8.

10. Composition pharmaceutique selon la revendication 9, dans laquelle ledit tampon est choisi parmi un citrate, un tartrate ou un acétate.

11. Composition pharmaceutique selon la revendication 9 ou la revendication 10, dans laquelle ledit agent stabilisateur est du mannitol.

12. Composition pharmaceutique selon l'une quelconque des revendications 9 à 11, dans laquelle ladite solution comprend en outre de l'eau.

13. Composition pharmaceutique selon l'une quelconque des revendications 9 à 12, dans laquelle ladite hormone parathyroïdienne humaine est choisie parmi PTH(I-34), PTH(I-37), PTH(I-38), PTH(I- 41) et PTH(I-84).

14. Composition pharmaceutique selon la revendication 13, dans laquelle ladite hormone parathyroïdienne humaine est PTH(I-34).

15. Flacon ou ampoule contenant une composition pharmaceutique non lyophilisée sous la forme d'une solution prête à être administrée par voie parentérale à un patient humain, ladite composition comprenant:
(a) de l'hormone parathyroïdienne humaine;
(b) un tampon destiné à maintenir un pH compris entre 3 et 7; et
(c) un agent stabilisateur

16. Flacon ou ampoule selon la revendication 15, dans laquelle ledit tampon est choisi parmi un citrate, un tartrate ou un acétate.

17. Flacon ou ampoule selon la revendication 15 ou la revendication 16, dans laquelle ledit agent stabilisateur est du mannitol.

18. Flacon ou ampoule selon l'une quelconque des revendications 15 à 17, dans laquelle ladite solution comprend en outre de l'eau.

19. Flacon ou ampoule selon l'une quelconque des revendications 15 à 18, dans laquelle ladite hormone parathyroïdienne humaine est choisie dans le groupe comprenant PTH(I-34), PTH(I-37), PTH(I-38), PTH(I-41) et PTH(I-84).

20. Flacon ou ampoule selon la revendication 19, dans laquelle ladite hormone parathyroïdienne humaine est PTH(I-34).

21. Flacon ou ampoule selon l'une quelconque des revendications 15 à 20, dans laquelle ladite hormone parathyroïdienne a une concentration comprise entre 25 µg/ml et 1000 µg/ml.

22. Composition pharmaceutique non lyophilisée selon l'une quelconque des revendications 1 à 6, ou l'une quelconque des revendications 9 à 14, destinée à être utilisée comme médicament.

23. Composition pharmaceutique non lyophilisée selon la revendication 22, destinée à être utilisée dans le traitement de l'ostéoporose.

24. Utilisation d'une composition pharmaceutique non lyophilisée selon l'une quelconque des revendications 1 à 6, ou l'une quelconque des revendications 9 à 14, dans la fabrication d'un médicament.

25. Utilisation selon la revendication 24, dans la fabrication d'un médicament permettant de traiter l'ostéoporose.
